(19) Europäisches Patentamt
European Patent Office
Office européen des brevets

(11) Veröffentlichungsnummer: **0 291 988 B1**

(12) **EUROPÄISCHE PATENTSCHRIFT**

(45) Veröffentlichungstag der Patentschrift: **25.11.92**

(51) Int. Cl.⁵: **C07K 17/08**, C12P 21/02, //C12N15/00

(21) Anmeldenummer: **88108057.6**

(22) Anmeldetag: **19.05.88**

# (54) IL-2 Reinigung mittels Rezeptor-Affinitäts-Chromatographie.

(30) Priorität: **19.05.87 US 51967**

(43) Veröffentlichungstag der Anmeldung:
**23.11.88 Patentblatt 88/47**

(45) Bekanntmachung des Hinweises auf die Patenterteilung:
**25.11.92 Patentblatt 92/48**

(84) Benannte Vertragsstaaten:
**AT BE CH DE FR GB IT LI NL**

(56) Entgegenhaltungen:
**EP-A- 0 147 819**
**EP-A- 0 210 645**

**JOURNAL OF IMMUNOLOGY, Band 136, Nr. 11, Juni 1986, Seiten 4099-4105, The American Association of Immunologists, US; B.F. TREIGER et al.: "A secreted form of the human interleukin 2 receptor encoded by an "anchor minus" cDNA"**

**BIOTECHNOLOGY, Band 5, Nr. 11, November 1987, Seiten 1195-1198, New York, US; P. BAILON et al.: "Receptor-affinity chromatography: A one-step purification for recombinant interleukin-2"**

(73) Patentinhaber: **F. HOFFMANN-LA ROCHE AG**
**Postfach 3255**
**CH-4002 Basel(CH)**

(72) Erfinder: **Bailon, Pascal**
**21 Woodbine Road**
**Florham Park, N.J. 07932(US)**
Erfinder: **Smart, John Edward**
**50 Meadowbrook Road**
**Weston Massachusetts 02193(US)**
Erfinder: **Weber, David Vincent**
**98 Hawthorne Avenue**
**Nutley, N.J. 07110(US)**

(74) Vertreter: **Lederer, Franz, Dr. et al**
**Patentanwalt Dr. Franz Lederer Lucile-Grahn-Strasse 22**
**W-8000 München 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die vorliegende Erfindung betrifft Methoden zur Reinigung von Interleukin-2. Lösliche Rezeptoren für Interleukin-2 wurden unter Verwendung der DNA-Rekombinationstechnologie hergestellt und an einem festen Träger immobilisiert. Die so hergestellten immobilisierten Rezeptoren wurden zur Herstellung von biologisch aktivem, nicht-aggregiertem, hoch reinem Interleukin-2 verwendet.

In den letzten Jahren sind eine grosse Anzahl wichtiger, biologisch aktiver Moleküle aus natürlichen Quellen gereinigt oder durch DNA-Rekombinationstechnologie hergestellt worden. Viele dieser Moleküle sind Polypeptide oder Proteine, die an spezifische zelluläre Rezeptoren binden.

Zur Reinigung dieser Polypeptide und Proteine wurden konventionelle Methoden verwendet wie z.B. präparative Elektrophorese, Gelfiltrationschromatographie, fraktionierte Fällung mit Salzen, Fraktionierung in organischen Lösungsmitteln, Ionenaustausch-Chromatographie und weitere bekannte Methoden. Problematisch bei der Verwendung von solchen konventionellen Methoden ist, dass diese im allgemeinen nicht für das gewünschte Protein spezifisch sind. Die Folge davon ist, dass manche dieser Methoden kombiniert werden müssen, bevor ein im wesentlichen reines Protein erhalten wird. Wegen der Verluste durch die Probenentnahme für die Analyse und wegen der unvollständigen Ausbeute bei jedem Reinigungsschritt, ist die Gesamtausbeute bei solchen mehrstufigen Reinigungsverfahren oft sehr klein.

Durch die Verwendung von Antikörperaffinitätssäulen mit polyklonalen oder monoklonalen Antikörpern, die spezifisch sind für ein zu reinigendes Protein, wurden in letzter Zeit bessere Resultate erhalten. Kung (U.S. Patent No. 4,476,049) z.B. hat monoklonale Antikörper verwendet, die spezifisch sind für das Carboxyterminale Ende von menschlichem Immuninterferon, um rekombinantes, menschliches Immuninterferon zu reinigen. Die Verwendung von Antikörperaffinitätssäulen kann zu hoch gereinigten oder sogar homogenen Protein-Präparationen führen, doch können Proteine, die auf diese Art und Weise gereinigt worden sind, unter Umständen nicht mehr ihre volle biologische Aktivität besitzen.

Pestka (U.S. Patent No. 4.623,621) hat gezeigt, dass manche wichtige, biologisch aktive Polypeptide und Proteine aggregieren und dabei Dimere, Trimere oder höhere oligomere Formen bilden. Solche Aggregate, können entweder eine geringere biologische Aktivität aufweisen, oder ihre Aktivität vollständig verloren haben. Zudem kann das aggregierte Material unerwünschte Nebenwirkungen verursachen, Z.B. zu Antikörperproduktion bei Patienten führen, denen es verabreicht wird. Antikörperaffinitätssäulen unterscheiden nicht notwendigerweise zwischen aggregierten und nicht-aggregierten (monomeren) Formen eines Polypeptids oder Proteins.

Die Wahrscheinlichkeit, dass ein bestimmtes Polypeptid oder Protein aggregiert, ist beträchtlich erhöhte, wenn es durch Verwendung von DNA-Rekombinationstechniken in Wirtsorganismen hergestellt und unter Bedingungen und in Konzentrationen isoliert wird, die nicht vorkommen, wenn es unter natürlichen Bedingungen aus Zell-Lysaten isoliert wird. Diese Bedingungen können eine Aggregation durch intermolekulare Disulfidbrücken, durch andere kovalente Bindungen oder durch nicht-kovalente Interaktionen begünstigen.

Weil die meisten, wenn nicht alle biologisch aktiven Polypeptide und Proteine dadurch wirken, dass sie an spezifische zellulärere Rezeptoren binden, wurden in den letzten Jahren zahlreiche Versuche unternommen, diese Rezeptoren zu charakterisieren. In letzter Zeit wurden Methoden der DNA-Rekominationstechnologie zur Herstellung von Rezeptormolekülen verwendet, um diese Rezeptormoleküle zu studieren. So wurden Gene, die für Rezeptoren für Interleukin-2 kodieren, kloniert und exprimiert.

Interleukin-2 (IL-2) ist ein Hormon-ähnlicher Proteinwachstumsfaktor, der durch gewisse T-Lymphozyten, nach antigener oder mitogener Stimulierung, sezerniert wird. Die Wirkung von IL-2 erfolgt über die Bindung des IL-2-Proteins an spezifische Rezeptoren mit hoher Affinität für IL-2. Diese Rezeptoren sind in den Membranen von aktivierten nicht aber von ruhenden Lymphozyten vorhanden.

Miller et al. [J. Immunol. 134, 4212-4217 (1985)] und Shimuzu et al. (Nucleic Acids Res. 13, 1505-1516 [1985]) haben eine cDNA, die für den IL-2-Rezeptor der Maus kodiert, kloniert und exprimiert. Leonard et al. [Nature 311, 626-631 (1984)] und Nikaido et al. [Nature 311, 631-635 (1984)] haben die Klonierung von cDNAs, die den menschlichen IL-2-Rezeptor kodierende beschrieben. In allen diesen Studien, wurden Plasmide verwendet, die ein IL-2-Rezeptor kodierendes Fragment enthielten, um COS-Zellen zu transfizieren. Mittels spezifischer Antikörper oder mittels IL-2-Bindung wurde gefunden, dass die transfizierten Zellen den exprimierten IL-2-Rezeptor in ihrer Zellmembran enthielten. In diesen Studien wurden keine Hinweise gefunden, dass Rezeptormoleküle in das Kulturmedium sezerniert worden waren.

Kürzlich haben Treiger et al. [J. Immunol.136, 4099-4105 (1986)] die Expression einer sezernierten Form des menschlichen IL-2-Rezeptors beschrieben. Diese sezernierte Form des Rezeptors wurde mittels eines Plasmids hergestellt, das eine mit NaeI geschnittene IL-2-Rezeptor-cDNA enthielt. Dabei wurde die Nukleinsäuresequenz, die für das intracytoplasmatische Segment und für den grössten Teil des mutmasslichen Transmembransegmentes ("Anker") des vollständigen Rezeptormoleküls kodiert, ausgeschnitten. Die resultierende modifizierte cDNA wurde dann in modifizierte Maus-L-Zellen transfiziert und exprimiert. Die Polypeptide, die dabei produziert wurden, wurden in das Medium sezerniert, weil ihnen der "Anker" fehlte.

Die vorliegende Erfindung betrifft IL-2-Rezeptor-Affinitätsadsorbenzien, die lösliche IL-2-Rezeptormoleküle, die auf einem festen Träger immobilisiert sind, enthalten und Verfahren zu deren Herstellung. Die Erfindung betrifft ferner Verfahren zur Reinigung von IL-2, worin solche Adsorbenzien verwendet werden. Diese Methoden können zur Reinigung von IL-2 beliebiger Herkunft verwendet werden, d.h. zur Reinigung von natürlichem, chemisch synthetisiertem oder mittels rekombinanter DNA-Expressionssysteme erhaltenem Material.

In einer Ausführungsform der Erfindung wird IL-2, das von menschlichen oder tierischen Zellen in Kultur produziert wurde, dadurch gereinigt, dass

(a) eine IL-2-haltige Lösung oder ein konditioniertes Medium, das IL-2 enthält, mit einem für IL-2 spezifischen Affinitätsadsorbens, das lösliche IL-2-Rezeptormoleküle, die auf einem festen (unlöslichen) Träger immobilisiert sind, enthält, in Kontakt gebracht wird;
(b) das Adsorbens zwecks Entfernung ungebundener, verunreinigender Substanzen gewaschen wird;
(c) das IL-2 vom gewaschenen Adsorbens desorbiert wird und
(d) das Rezeptor-Affinitätsadsorbens vom desorbierten IL-2 abgetrennt wird.

In einer anderen Ausführungsform der Erfindung wird rekombinantes IL-2, das in einem bakteriellen Expressionssystem hergestellt worden ist, dadurch gereinigt, dass:

(a) kultivierte, bakterielle Zellen, die mit einem Vektor transformiert sind, der fähig ist, IL-2 zu exprimieren, aufgebrochen werden und ein Zell-Lysat hergestellt wird;
(b) die unlösliche Fraktion des Zell-Lysats isoliert wird;
(c) die isoliert, unlösliche Fraktion des Zell-Lysats mit einer Lösung von etwa 4-7 M Guanidin•HCl extrahiert wird, wodurch ein IL-2 enthaltender Extrakt erhalten wird;
(d) dieser Extrakt mit einer Guanidin•HCl-freien Lösung etwa 10- bis 1000-fach verdünnt wird;
(e) der verdünnte Extrakt mit einem für IL-2 spezifischen Affinitätsadsorbens, das lösliche IL-2-Rezeptormoleküle, die auf einem festen (unlöslichen) Träger immobilisiert sind, enthält, in Kontakt gebracht wird;
(f) das Adsorbens zwecks Entfernung ungebundener, kontaminierender Substanzen gewaschen wird;
(g) das IL-2 vom gewaschenen Adsorbens desorbiert wird und
(h) das Rezeptoraffinitätsadsorbens vom desorbierten IL-2 abgetrennt wird.

Obwohl der Grad der Verdünnung in Schritt (d) etwa 10- bis etwa 1000-fach sein kann, ist eine Verdünnung des Extrakts von etwa 30- bis etwa 50-fach bevorzugt.

In einer weiteren Ausführungsform der Erfindung wird rekombinantes IL-2, das in bakteriellen Expressionssystemen hergestellt worden ist, wie oben beschrieben gereinigt, mit der Ausnahme, dass dem Extraktionsschritt (c) ein Präextraktionsschritt vorausgeht, in dem kontaminierendes, zelluläres Material extrahiert wird. Der Präextraktionsschritt umfasst die Extraktion der isolierten unlöslichen Fraktion des Zell-Lysats mit

(i) einer gepufferten Salzlösung und
(ii) einer Lösung von etwa 1,75 bis 2 M Guanidin•HCl.

In einer weiteren Ausführungsform der Erfindung wird rekombinantes IL-2 in einem bakteriellen Expressionssystem hergestellt und wie oben beschrieben gereinigt, mit der Ausnahme, dass auf den Verdünnungsschritt (d) ein Schritt folgt, bei dem der verdünnte Extrakt während einer Zeitdauer von 4 Stunden bis zu mehr als 3 Tagen stehengelassen wird. Vorzugsweise wird der verdünnte Extrakt während etwa 1 bis 3 Tagen stehengelassen.

Die nachfolgende, detaillierte Beschreibung und die nachfolgenden Figuren illustrieren die vorliegende Erfindung.

Fig. 1 ist eine schematische Abbildung des Prinzips der IL-2-Rezeptor-Affinitätschromatographie. IL-2 wird in Schritt I spezifisch an die Matrix M (Gelkugeln) mit den immobilisierten IL-2-Rezeptormolekülen (IL-2R) gebunden. Die kontaminierenden Proteine P werden durch den Wasch-Schritt II entfernt. Das IL-2 wird in Schritt III von der Matrix desorbiert, was regenerierte Gelkugeln und gereinigtes IL-2 ergibt.

Fig. 2 zeigt ein Sephadex® G-50-Säulen-Elutionsprofil von rekombinantem, menschlichem IL-2, das in E. coli hergestellt worden ist und durch Rezeptor-Affinitätschromatographie gereinigt wurde. Abszisse: Fraktionen (F.); Ordinate: Absorption bei 280 nm ($A_{280}$).

Fig. 3 zeigt ein Sephadex® G-50-Säulen-Elutionsprofil von rekombinantem, menschlichem IL-2, das in Säugerzellen hergestellt worden ist und durch Rezeptor-Affinitätschromatographie gereinigt wurde. Abszisse: Fraktionen (F.); Ordinate: Absorption bei 280 nm ($A_{280}$).

Fig. 4 zeigt ein Sephadex® G-50-Säulen-Elutionsprofil von rekombinantem menschlichem IL-2, das in

E. coli hergestellt worden ist und durch Antikörper-Affinitätschromatographie gereinigt wurde. Abszisse: Fraktionen (F.); Ordinate: Absorption bei 280 nm ($A_{280}$).

Fig. 5 zeigt das Resultat einer SDS-Polyacrylamid-gelelektrophoretischen Analyse folgender Proben unter nicht-reduzierenden Bedingungen: Mischung von Standard-Markierproteinen (Spur S); Extrakt eines rekombinanten E. coli (Spur 1); Durchfluss der Reinigung des E. coli-Extrakts in der Rezeptoraffinitätssäule (Spur 2); am Rezeptoraffinitätsadsorbens gereinigtes rekombinantes IL-2 aus dem E. coli-Extrakt (Spur 3); konditioniertes Medium von transfizierten Säugerzellen, die IL-2 Produzieren (Spur 4); und am Rezeptoraffinitätsadsorbens gereinigtes rekombinantes IL-2 aus einem konditionierten Medium (Spur 5). Die Zahlen auf der rechten Seite des Gels bezeichnen die Molekulargewichte der Markierproteine in Kilodalton (= 1'000 Dalton);

Fig. 6 zeigt das Resultat einer SDS-Polyacrylamid-gelelektrophoretischen Analyse folgender Proben unter reduzierenden (R) und nicht-reduzierenden (NR) Bedingungen:
Gemisch von Standard-Markierproteinen (Spur S); E. coli-Extrakt enthaltrend [des-$Ala^1$-$Ser^{125}$]-IL-2 (Spur 1); am Affinitätsadsorbens gereinigtes [des-$Ala^1$-$Ser^{125}$]-IL-2 (Spur 2); E. coli-Extrakt enthaltend [$Lys^{20}$]-IL-2 (Spur 3); am Affinitätsadsorbens gereinigtes [$Lys^{20}$]-IL-2 (Spur 4); E. coli-Extrakt enthaltend Wildtyp rekombinantes, menschliches IL-2 (Spur 5); und Affinitätsadsorbens gereinigtes Wildtyp rekombinantes, menschliches IL-2 (Spur 6). Die Zahlen auf der linken Seite des Gels bezeichnen die Molekulargewichte der Markierproteine in Kilodalton (=1'000 Dalton).

Mit der vorliegenden Erfindung wird die stark verbesserte Reinigung von IL-2 dadurch erreicht, dass ein Rezeptor-Affinitätschromatographie-Verfahren durchgeführt wird. Das Verfahren ist schematisch in Fig. 1 dargestellt. In dem Verfahren wird ein bakterieller Extrakt oder eine IL-2 haltige Lösung bzw. ein konditioniertes Medium, worin IL-2 enthalten ist, mit einem unlöslichen Träger, an den gereinigte, lösliche Rezeptoren für IL-2 (IL-2R) immobilisiert worden sind, kontaktiert. Die IL-2R-Moleküle, die an die Gelkugeln fixiert worden sind, sind in Fig. 1 als Pfeile dargestellt.

Weil nur IL-2 in dem Extrakte, der Lösung oder dem Medium eine spezifische, hohe Affinität für die immobilisierten Rezeptoren hat, kann nur IL-2 an die Kugeln binden (oder an sie adsorbieren). Die Konsequenz davon ist, dass alle verunreinigenden (kontaminierenden) Proteine, denen eine solche hohe Affinität fehlte, ungebunden bleiben und rasch ausgewaschen werden. Das IL-2, das spezifisch an die Kugeln gebunden ist, kann durch geeignete Mittel desorbiert werden, mit dem Resultat, dass regenerierte Kugeln und gereinigtes IL-2 erhalten wird.

Das IL-2, das mittels der erfindungsgemässen Verfahren gereinigt werden kann, kann aus verschiedenen Quellen stammen. Z.B. kann es chemisch durch Flüssig- oder Festphasenmethoden synthetisiert werden. Die Aminosäuresequenz von Human-IL-2 siehe bei Taniguchi et al., Nature 302, 305-310 (1983), diejenige von Maus-IL-2 ist von Yokota et al. in Proc. Natl. Acad. Sci. USA 82, 68-72 (1985), publiziert worden. IL-2 kann auch in bakteriellen oder Säugerzell-Expressionssystemen hergestellt werden, wobei die Methoden der DNA-Rekombinationstechnologie, unter Berücksichtigung der bekannten Sequenz des IL-2-Gens, verwendet werden.

Transformierte Bakterien, die rekombinantes IL-2 produzieren, müssen im allgemeinen mittels bekannter Methoden aufgebrochen werden. Beispiele für solche Methoden sind Ultraschallbehandlung, Zellaufbrechung mittels mechanischer Mittel, z.B. mit einer Frenchpresse, durch osmotischen Schock oder durch wiederholtes Gefrieren und Tauen.

Kulturen von Lymphozyten oder T-Zellinien können ebenfalls als Quelle für IL-2 dienen, Z.B. haben Gillis et al., J. Exp. Med. 152, 1709-1719 (1980) die Herstellung von IL-2 in konditioniertem Medium von kultivierten Lymphozyten der Maus, der Ratte oder des Menschen oder von T-Zellinien von leukämischen Mäusen oder von menschlichen T-Zellinien, beschrieben. Solche kultivierten Zellen werden im allgemeinen, durch Agenzien, wie z.B. Phytohämagglutinin oder Concanavalin A, dazu stimuliert, IL-2 zu produzieren. Die Rezeptor-Affinitätsadsorbenzien der vorliegenden Erfindung sind, wegen ihrer Fähigkeit selektiv IL-2 aus grossen Volumen von Medium abzutrennen, speziell geeignet, IL-2 aus solchen konditionierten Medien zu reinigen.

Die IL-2-Rezeptoren der vorliegenden Erfindung können beliebige Rezeptoren sein, die spezifisch für lösliches IL-2 sind. In der bevorzugten Ausführungsform der Erfindung, wird eine Modifikation des menschlichen IL-2-Rezeptors, der der Anker fehlt ("anchor minus" modification), wie sie von Treiger et al., supra, beschrieben ist, verwendet. Dieses Molekül ist löslich, weil die Carboxy-terminalen 28 Aminosäurereste entfernt worden sind. Dadurch werden die Moleküle nicht in den Membranen von IL-2R produzierenden Zellen zurückgehalten. Weil das gesamte menschliche IL-2R-Gen kloniert und sequenziert worden ist [Cosman et al., Nature 312, 768-771 (1984)], können Varianten, die mehr oder weniger Aminosäurereste enthalten, und die die erforderlichen Charakteristika haben, mittels bekannter Methoden der organische

Synthese oder der DNA-Rekombinationstechnologie, hergestellt werden.

Da auch Sequenzdaten der IL-2-Rezeptoren der Maus verfügbar sind [Miller et al., supra], können lösliche Formen dieser Rezeptoren in analoger Weise wie uie menschlichen Rezeptoren hergestellt werden.

Zwischen der Aminosäuresequenz des IL-2R der Maus und derjenigen des menschlichen IL-2R besteht eine 61%ige Homologie [Miller et al., supra]. Daher kann vermutet werden, dass die IL-2-Moleküle der einen Art mit den IL-2-Rezeptoren der andern Art kreuzreagieren. Tatsache ist, dass rekombinantes IL-2 der Maus an menschliches IL-2R-Rezeptoraffinitätsadsorbens bindet und demzufolge mittels einem solchen Adsorbens gereinigt werden kann.

Jegliche Rezeptoren, die in der vorliegenden Erfindung verwendet werden, sollten folgende Charakteristika aufweisen:

1. Sie sollten löslich sein, um die Reinigung, vorzugsweise bis zur Homogenität, zu erleichtern.
2. Die Gene, die sie kodieren, sollten klonierbar sein und ein hohes Mass an Expression ergeben, sodass beträchtliche Mengen des Genproduktes erhalten werden können.
3. Auch an einem geeigneten Trägermaterial immobilisiert, sollten sie noch IL-2 erkennen und mit hoher Affinität binden.

Beispiele für feste Träger, die verwendet werden können, sind Agarosegele; quervernetzte Agarosegele; Glaskugeln mit kontrollierter Porengrösse (controlled-pore glass beads), vorzugsweise mit Glycerin- oder Glykol-ähnlichen Strukturen beschichtet, um unspezifische Proteinadsorption zu verhindern; Cellulosepartikel; Polyacrylamidgelkugeln und quervernetzte Dextrane, wie z.B. Sephadex®. Ein modifiziertes Polyhydroxy-Kieselgelträgermaterial, wie im folgenden beschriebenen, ist bevorzugt.

Der erfindungsverwendete, lösliche IL-2-Rezeptor kann einerseits direkt an ein festes Trägermaterial gebunden sein, oder kann andererseits über eines der zahlreichen Verbindungsmoleküle, die bekannt sind, an einen Träger gebunden sein. Einige Beispiele solcher Verbindungsmoleküle, die verwendet werden können, wurden, z.B. von Lowe et al. in Int. J. Biochem. 13, 33-40 (1981), beschrieben.

Die Selektion eines Mittels zur kovalenten Bindung von IL-2-Rezeptoren an ein unlösliches Trägermaterial ist abhängig von der Natur des verwendeten Trägermaterials. Für Träger auf der Basis von Zuckern, wie z.B. Agarose oder Cellulose, kann die Reaktion mit Bromcyan in wässrigem Alkali verwendet werden, um ein aktiviertes Trägermaterial herzustellen, an das Proteine oder Polypeptide unmittelbar gekoppelt werden können. Bromcyan aktivierte Agarose ist käuflich erhältlich. Bisoxirane und Epihalohydrine können ebenfalls für diese Zwecke verwendet werden [Porath et al., J. Chromatogr. 60, 167-177 (1971); Nishikawa et al., J. Solid-Phase Biochem. 1, 33-49 (1976)].

Abgewandelte Polyacrylamidgele können durch Behandlung mit Hydrazin hergestellt werden, worauf dann eine Vielzahl von Acylierungs- und Alkylierungsreagenzien zur weiteren chemischen Umwandlung verwendet werden können [Inman et al., Biochemistry 8, 4074-4082 (1969)].

Glas mit kontrollierter Porengrösse kann mit γ-Aminopropyltriethoxysilan behandelt werden, um Silanolgruppen, die zu unspezifischer Adsorption von Proteinen führen, abzudecken. Der γ-Aminopropylteil liefert Anknüpfungspunkte, an die IL-2-Rezeptoren gebunden werden können. Derivate von Glycerin- oder Glykolbeschichteten Glaskugeln können durch Behandlung mit Natriumperjodat erhalten werden [Sanderson et al., Immunochem. 8, 163-168 (1971)].

Für eine allgemeinen Uebersicht über Kupplungsreaktionen siehe Weliky et al., Immunochem. 2, 293-322 (1965) und Guilford, Chem. Soc. Rev. 2, 249-270 (1973).

Das erfindungsgemässe IL-2-Rezeptor-Affinitätsabsorbens kann in säulenchromatographischen Verfahren, wie sie unten beschrieben sind, oder in Eintopfverfahren verwendet werden. Eintopfverfahren werden vorzugsweise verwendet, wenn das Adsorbens im Säulenbetrieb niedrige Durchflussraten liefert oder zu Verstopfungen führt, oder wenn lange Kontaktzeiten nötig sind, um eine vollständige IL-2-Bindung sicherzustellen.

Im Eintopfverfahren wird der immobilisierte IL-2R einfach durch Rühren oder Schütteln in der das IL-2 enthaltenden Lösung bzw. dem Extrakt oder konditionierten Medium, während einer gewünschten Zeit, suspendiert. Danach wird das Adsorbens durch Filtration oder Sedimentation, durch Dekantieren oder durch Absaugen der flüssigen Phase, nachdem das Adsorbens sich gesetzt hat, abgetrennt. Das Adsorbens kann dann gewaschen werden und das gebundene IL-2 kann anschliessend desorbiert werden.

Eine Variante des Eintopfverfahrens, das sogenannte Teebeutelverfahren, kann ebenfalls verwendet werden. Bei diesem Verfahren ist das IL-2-Rezeptor-Affinitätsadsorbens in einem durchlässigen Beutel, der aus Stoff oder einem anderen Material gemacht sein kann, enthalten. Der Beutel wird dann während einer gewissen Zeit in den IL-2 enthaltenden Extrakt, die Lösung oder das konditionierte Medium eingetaucht, aus der er dann wie ein Teebeutel entfernt wird. Die Vorteile dieser Methode sind die Leichtigkeit der Abtrennung des Adsorbens und die vereinfachte Aufarbeitung.

Ist das IL-2 einmal an das IL-2-Rezeptoraffinitätsadsorbens gebunden, wird das Adsorbens gewaschen,

um verunreinigendes Material zu entfernen. Anschliessend wird das IL-2 vom Adsorbens gelöst. In einer bevorzugten Ausführungsform der Erfindung wird dazu eine Desorptionslösung mit tiefem pH, die 0,2 N Essigsäure und 0.2 M NaCl enthält, verwendet. Im allgemeinen können Wechsel im pH, der Ionenstärke oder der Lösungsmittelzusammensetzung (z.B. durch Zugabe von wassermischbaren, organischen Lösungsmitteln oder chaotropen Agenzien) zur Lösung des IL-2 vom Adsorbens verwendet werden. Solche Aenderungen verringern die Stärke der Interaktion zwischen dem IL-2 und dem immobilisierten IL-2R bis zu einem Punkt, bei dem das IL-2 abgelöst (desorbiert) wird. Die Desorptionsbedingungen sind so zu wählen, dass weder das IL-2 noch der IL-2R irreversibel denaturiert werden, letzteres damit das Absorbens wiederholt verwendet werden kann.

## Beispiele

### 1. Allgemeine Methoden

Falls nicht anders angegeben, wurden die Proteinbestimmungen gemäss der Methode von Lowry et al., J. Biol. Chem. 193, 265-275 (1951), unter Verwendung von Rinderserumalbumin als Standard, durchgeführt. Die dabei erhaltenen Werte wurden durch quantitative Aminosäureanalyse bestatigt. Die Aminosäureanalyse wurde gemäss Pan et al., Methods of Protein Microcharacterization (Shively, J.E., Herausgeber), The Humana Press Inc., Clifton, New Jersey, U.S.A., S. 105 (1986) durchgeführt, nachdem die Proben in 6 N Salzsäure, die 4% Thioglykolsäure enthielt, bei 110 °C während 20-24 Stunden im Vakuum hydrolysiert worden sind. Die Werte für Prolin und Cystein wurden nach Oxidation in Perameisensäure bestimmt. Natriumdodecylsulfat (SDS) Polyacrylamidgelelektrophorese wurde in 12% Gelen, wie von Laemmli, Nature 227, 680-685 (1970) beschrieben, durchgeführt.

Das IL-2-Testverfahren wurde unter Verwendung einer kolorimetrischen Modifikation der Methode von Gillis et al., J. Immunol. 120, 2027-2032 (1978) durchgeführt. Bei diesem Testsystem werden CTLL-Zellen, das sind Zellen einer zytotoxischen Lymphozytenzellinie der Maus, die, bezüglich des Wachstums, IL-2 abhängig sind [Gillis et al., J. Exp. Med. 146, 468-482 (1977)], als Zielzellen verwendet. CTLL-Zellen sind erhältlich bei der American Type Culture Collection, 12301 Parklawn Drive, Rockville, Maryland 20852, U.S.A. (CTLL-2, ATCC TIB 214) oder von Immunex Corp., Seattle, Washington, U.S.A. Die CTLL-Zellen wurden in RPMI-1640 Medium (Gibco Laboratories, Grand Island, New York, U.S.A.), das mit 5% (v/v) fötalem Kälberserum (Gibco), 25 mM HEPES-Puffer, 50 µg/ml Gentamicin (Schering/Plough Inc., Bloomfield, New Jersey, U.S.A.) und 200 Einheiten/ml rohem, menschlichem IL-2 ergänzt war, gezüchtet. Als Alternative können auch menschliche PHA-Blastzellen als Zielzellen verwendet werden. Menschliche PHA-Blastzellen können durch Stimulierung von menschlichen peripheren Blutlymphozyten mit dem Mitogen Phytohämagglutinin (PHA) während mindestens 5 Tagen erhalten werden.

Für das Testverfahren wurden CTLL-Zellen aus dem IL-2 enthaltenden Kulturmedium, durch Sedimentation während 10 Minuten bei 500 x g in einer Beckman Model J-6B-Zentrifuge, entfernt und zweimal in HB101-Medium (Hana Biologicals, Berkeley, California, U.S.A.) ohne IL-2 gewaschen. Die Zellen wurden in HB101 das mit 50 µg/ml Gentamicin ergänzt worden war, resuspendiert und auf eine Konzentration von 2 x $10^5$ Zellen/ml eingestellt. Proben, die zu titrieren waren, wurden in HB101 in den ersten Vertiefungen einer Reihe einer Mikrotiterplatte (Costar Nr. 3696) auf ein Endvolumen von 0,1 ml verdünnt. Die nachfolgenden Vertiefungen in der Reihe enthielten 0,05 ml HB101. Zweifache Verdünnungen der Proben wurden durch Uebertragung von 0,05 ml Proben bis zum Ende der Reihen erhalten. Einige Verdünnugen, die mit 0,05 ml Medium ohne IL-2 gefüllt waren, dienten als negative Kontrollen. Alle Vertiefungen wurden mit 0,05 ml der Zellsuspension beschickt. Die Platten wurden mit einem Plastikdeckel bedeckt und während 20 Stunden bei 37 °C in einem befeuchteten 5% $CO_2$-Raum inkubiert.

Das Testverfahren basiert auf der kolorimetrischen Bestimmung von Milchsäure, die als Endprodukt des Glucosemetabolismus durch stimulierte Zellen Produziert wird. Die Reagenzien für die kolorimetrische Bestimmung von Milchsäure wurden von der Sigma Chemical Co., St. Louis, Missouri, U.S.A. erhalten. Lösung A enthielt 1,6 mg/ml ß-Nikotinamid-adenin-dinukleotidund 0,03% (w/v) Gelatine in einem Puffer aus 0.6 M Glycin, 80 mM Hydrazin, bei einem PH von 9,2. Lösung B enthielt 166 Einheiten/ml Lactatdehydrogenase, 0.5 mg/ml p-Jodnitrotetrazoliumviolett und 0,03 mg/ml Phenazin Methosulfat in Wasser. Eine Milchsäurestandardlösung, die mit phosphatgepufferter Salzlösung verdünnt war, diente als positive Kontrolle für das Testverfahren. Eine Lösung mit 0.35 N HCl wurde zum Beenden der Reaktion verwendet. Eine Mikrotiterplatte mit 96 Vertiefungen (Dynatech), die 0.05 ml Lösung A in jeder Vertiefung enthielt, wurde als Reaktionsplatte verwendet, um die Menge Milchsäure in einer 0,015 ml-Probe, die von entsprechenden Vertiefungen der biologischen Testmikrotiterplatte erhalten worden war, zu bestimmen.

Durch die Zugabe von 0,05 ml Lösung B zu jeder Vertiefung in der Reaktionsplatte wurde die Reaktion

gestartet. Nach einer Zeit, die genügte um den Tetrazolfarbstoff zu reduzieren, wurde die Reaktion durch Zugabe von 0,05 ml HCl-Lösung zu jeder Vertiefung gestoppt. Die optische Dichte bei 550 nm jeder Vertiefung der Reaktionsplatte wurde mit einem Mikroplattenspektrophotometer (Artek Model 200) bestimmt.

Eine Einheit IL-2-Aktivität ist definiert als diejenige Menge IL-2, die eine halb-maximale Antwort in dem Testverfahren ergibt. Die IL-2-Konzentration ergibt sich aus dem Umkehrwert der Verdünnung in der 50% Endpunkt Vertiefung. Das Referenzreagens menschliches IL-2 (Jurkat) Material des National Institutes of Health Bureau of Biological Response Modifiers (BRMP) wurde als Standard verwendet.

IL-2-Rezeptorenmessungen wurden unter Verwendung der doppelten Epitop-ELISA-Methode von Rubin et al., J. Immunol. 135, 3172-3177 (1985) durchgeführt. Einheiten der IL-2-Rezeptoraktivität wurden definiert wie von Rubin et al. (supra) beschrieben.

2. Herstellung von Interleukin-2

Nicht-glycosyliertes, reifes, rekombinantes, menschliches IL-2 wurde gemäss bekannter Methoden in transformierten E. coli-Zellen hergestellt, während glycosyliertes, rekombinantes, menschliches IL-2 in transfizierten Säugerzellen hergestellt wurde [Taniguchi et al., Nature 302, 305-310 (1983); Europäische Patentanmeldung, Publikationsnummer 91539, publiziert am 19. Oktober 1983]. IL-2 von Lysaten von bakteriellen Zellen und aus Kulturmedium, das durch Säugerzellen konditioniert worden war, wurde wie unten beschrieben gereinigt.

In einer bevorzugten Ausführungsform der Erfindung wird rekombinantes IL-2 in E. coli hergestellt. Dabei wird unter Verwendung von synthetischen Oligonukleotiden, die auf der DNA-Sequenz von Taniguchi et al. [Nature 302, 305-310 (1983)] basieren, ein cDNA-Klon isoliert, der für menschliches IL-2 kodiert. Der cDNA-Klon wird dann verwende, um das Expressionsplasmid pRC 233/IL 2/Δtet zu konstruieren. Dieses Plasmid und die Methoden für seine Konstruktion sind von Ju et al. in J. Biol. Chem. 262, 5723-5731 (1987) ausführlich beschrieben. Transformanten, die dieses Plasmid enthalten, wurden unter Standardbedingungen wachsen gelassen.

In einer weiteren bevorzugten Ausführungsform der Erfindung, wurde rekombinantes IL-2 in einer Ovarzellinie des chinesischen Hamsters, der die Dihydrofolatreduktase fehlt ($CHO/dhfr^-$), und die mit dem menschlichen IL-2-Gen transfiziert ist, hergestellt. Das Gen wurde in den eukaryotischen Expressionsvektor pBC12MI eingeführt, der am 7. Mai 1986 in Form eines E. coli MC1061, der dieses Plasmid enthält, bei der American Type Culture Collection in Uebereinstimmung mit dem Budapester Vertrag, unter der Hinterlegungsnummer ATCC No. 67109, hinterlegt worden war. Der Vektor pBC12/RSV/IL-2/dhfr, der auf diese Art und Weise erhalten wurde, wurde in CHO-Zellen transfiziert und das IL-2-Gen wie in der deutschen Patentanmeldung P3715808.2, die am 10. Dezember 1987 offengelegt wurde, beschrieben, exprimiert.

3. Herstellung und Reinigung von rekombinanten löslichen IL-2-Rezeptoren

Eine sezernierte Form des menschlichen IL-2-Rezeptors wurde in Säugerzellen, gemäss allgemein bekannter Methoden, hergestellt [Treiger et al., supra]. Das Medium von kultivierten Zellen wurde gesammelt, und der darin enthaltene sezernierte IL-2-Rezeptor wurde daraus gereinigt.

In einer bevorzugten Ausführungsform der Erfindung, wurde das Plasmid pIL2R3, das von Treiger et al. [supra] beschrieben ist, mit dem Restriktionsenzym Nae I geschnitten, um ein 879 Basenpaare langes IL-2R-Genfragment auszuschneiden, das die gesamte 816 Basenpaare lange IL-2R-kodierende Sequenz enthält und die flankiert ist von 24 Basenpaaren der 5′- und 39 Basenpaaren der 3′-nichtkodierenden Sequenz. Die IL-2R cDNA wurde, unter Verwendung von Klenow-DNA-Polymerase, stumpf geschnitten und unter Verwendung von T4-DNA-Ligase zwischen stumpfe Hind III und Bam HI Stellen des oben beschriebenen Expressionsvektors pBC12MI eingebaut. Der auf diese Weise erhaltene Expressionsvektors, der das gewünschte IL-2R cDNA-Fragment in der richtigen Orientierung enthält, wurde mit pBC12/IL-2R bezeichnet.

Der Vektor pBC12/IL-2R Nae wurde durch Schneiden des Vektors pBC12/IL-2R an der einzigen Nae I-Stelle des IL-2R-Gens und an der einzigen Sma I Stelle des Vektors, gefolgt vom Einbau eines 14 Basenpaare langes Oligonukleotids, das Terminationscodons in allen drei Leserastern enthält (5′-TTAAGTTAACTTAA-3′), erhalten. Der Vektor pBC12/IL-2R Nae kodiert ein verkürztes IL-2R-Gen, dem die 28 Carboxy-terminalen Aminosäuren fehlen (ähnlich dem Gen von Treiger et al., supra) und enthält zusätzlich Codons für Leucin und Serin (5′-TTAAGT-3′).

Das Plasmid pBC12/IL-2R Nae wurde verwendet um $CHO/dhfr^-$-Zellen, gemäss der Methode von Cullen [Cell 46, 973-982 (1986)] zu transfizieren. Konditioniertes Medium von Kulturen solcher transfizierter Zellen enthält rekombinantes IL-2R.

Das konditionierte Medium der transfizierten CHO-Zellen wurde mittels Diafiltration in einem Millipore

Pellicon®-System zehnfach konzentriert. Dabei wurde eine PTGC-Kassette verwendet, die Molelüle mit mehr als 10,000 Dalton Molekulargewicht zurückhält. Der konzentrierte Ueberstand (1200 ml) wurde auf eine IL-2-Affinitatssäule (4,4 x 6,5 cm) aufgetragenen, die nichtglycosyliertes, reifes, rekombinantes, menschliches IL-2 enthielt, das, mit einer Kopplungsdichte von 4,7 mg/ml Gel, kovalent an Polyhydroxy NuGel-AF® Trägermaterial (Separation Industries, Metuchen, New Jersey, U.S.A.) gebunden ist. Polyhydroxy NuGel-AF® ist ein modifiziertes Polymeres Silikat das 3-[3-(2,3-Dihydroxy)propoxy]propyl-Gruppen der Formel

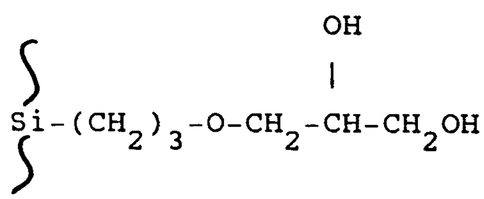

enthält. Das feste Trägermaterial wurde zuerst mit Perjodat oxidiert. Das IL-2 wurde dann, durch reduktive Aminierung in Gegenwart von Natriumcyanborhydrid, wie von Roy et al., J. Chromatogr. 303, 225-228 (1984) beschrieben, an das feste Trägermaterial gebunden.

Nichtadsorbiertes, verunreinigendes Material wurde mittels einer Waschung mit phosphatgepufferter Salzlösung (PBS) entfernt, und der spezifisch gebundene IL-2R wurde mit 3M KSCN in PBS desorbiert. Nach einer Dialyse gegen PBS wurde das Eluat der Affinitätssäule auf eine 4,4 x 20,5 cm DEAE-Silikatsäule (Nu Gel P-DE 200®, Separation Industries) aufgetragen. Der IL-2R wurde mit 0,2M NaCl in PBS eluiert. Eine stark gebundene Hauptverunreinigung eluierte in einem nachfolgenden 1M NaCl Elutionsschritt.

Eine Analyse mittels SDS-Polyacrylamid-Gelelektrophorese unter reduzierenden und nichtreduzienenden Bedingungen zeigte, dass der gereinigte IL-2R aus einer Mischung von einem Monomer und einem reduzierbaren Dimer besteht. Diese zwei Formen von IL-2R wurden mittels Gelfiltration an Sephacryl® S-200 (Pharmacia Fine Chemicals, Piscataway, New Jersey, U.S.A.), unter Verwendung von PBS als Elutionsmittel, abgetrennt. Die monomere Form des gereinigten IL-2R hatte eine spezifische Aktivität von $3{,}5 \times 10^8$ Einheiten/mg Protein.

4. Immobilisierung des IL-2-Rezeptors

Der gereinigte, monomere IL-2R wurde auf NuGel P-AF® Poly-N-hydroxysuccinimid (PNHS; 500 Å 40-60 µm; Separation Industries, Metuchen, New Jersey, U.S.A.) immobilisiert. NuGel P-AF® Poly-N-hydroxysuccinimid ist ein modifiziertes polymeres Silikat mit 3-[2-[[[2-[2-[(2,5-Dioxo-1-pyrrolidinyl)oxy] -2-oxoethyl]amino]ethoxy]propyl-Gruppen der Formel

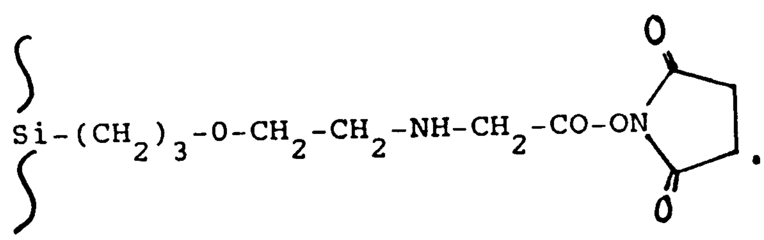

Das folgende Verfahren wurde verwendet. 20 g getrocknetes PNHS (entsprechend 28 ml gequollenes Gel) wurden in einen 150 ml grob gesinterten Glastrichter überführt und mit dreimal 28 ml kaltem Wasser gewaschen. Das gewaschene Gel wurde quantitativ in einen verschliessbaren 100 ml Erlenmeyerkolben transfiziert, der 28 ml einer 2,1 mg/ml Lösung von IL-2R in 0,1 M Kaliumphosphat mit 0,1 M NaCl, pH 7,0, enthielt. Die Mischung wurde während 16 Stunden bei 4°C sanft geschüttelt. Danach wurden nichtgekoppelte IL-2R-Moleküle durch Filration der Reaktionsmischung gesammelt. Das Gel wurde mit zweimal 28 ml PBS gewaschen. Das Filtrat und die Waschlösungen wurden kombiniert und über Nacht bei 4°C gegen 2 l PBS dialysiert.

Unmittelbar nach dem Waschen wurde das Gel mit 28 ml 100 mM Ethanolamin-HCl PH 7,0 vereinigt und während 1 Stunde geschüttelt, um jegliche nichtreagierte NHS-Estergruppen zu neutralisieren. Das IL-2R-Gel wurde dreimal mit 28 ml PBS gewaschen und dann im gleichen Puffer in Gegenwart von 0.1% Natriumazid bei 4° C gelagert.

Das Dialysatvolumen wurde dann gemessen und der Proteinwert spektrophotometrisch bei 280 nm, basierend auf einer optischen Dichte bei dieser Wellenlänge von 1,65 für eine 1 mg/ml IL-2R-Lösung, bestimmt. Berechnungen, die auf der Differenz zwischen der verwendeten Ausgangsmenge von IL-2R (58,8 mg) und der Menge von nicht-gekoppeltem IL-2R, das im Dialysat erhalten wurde (29,4 mg), beruhten, zeigten, dass die Kopplungsdichte von IL-2R 1,05 mg Protein/ml gequollenes Gel betrug.

5. Reinigung von rekombinantem IL-2 aus E. coli

E. coli-Zellen, die mit einem Plasmid transformiert worden sind, das das menschliche IL-2-Gen enthält, wurden gleichmässig in 30 mM Tris-HCl, pH 8,0, enthaltend 5 mM Ethylendiamintetraessigsäure (EDTA) [Puffer A, 4 ml/g Zellen] resuspendiert und bei 2-8° C gerührt. Die Suspension wurde zwei- oder dreimal durch einen Gaulin-Homogenisator (APV Gaulin, Everett, Massachusetts, U.S.A.) bei 7,000 psi (entsprechend etwa 4,8 x $10^7$ Pascal) geführt, wobei die Homogenisatorausgangstemperatur mit einer doppelten Hitzetransferschlange bei 2-8° C gehalten wurde.

Das Zellysat wurde bei 24,000 x g während 1 Stunde in einer Sorvall RC-5B-Zentrifuge mit einem GSA-Rotor (Dupont, Wilmington, Delaware, U.S.A.) zentrifugiert. Der Ueberstand, der lösliche Proteine enthielt, wurde verworfen. Das Sediment wurde in Puffer A (4 ml/g Zellen) resuspendiert und während 30-60 Minuten bei 4° C gerührt. Nach einem Zentrifugationsschritt, wie er oben beschrieben ist, wurde der Ueberstand verworfen.

Der Niederschlag wurde in 1,75 M Guanidin•HCl (4 ml/g) resuspendiert und während 30 Minuten bis 1 Stunde, wie oben beschrieben, vermischt. Der Ueberstand wurde verworfen. Das Sediment wurde in 7 M Guanidin•HCl (4 ml/g Zellen) resuspendiert und während 1 Stunde gerührt. Der Extrakt wurde wie oben beschrieben zentrifugiert und der resultierende Ueberstand wurde 40-fach mit PBS verdünnt. Der verdünnte Extrakt wurde während 24 Stunden bei 4° C stehengelassen. Während dem Stehenlassen der Lösung entwickelten sich flockenartige Partikel. Der verdünnte Ueberstand wurde sorgfälig von den ausgeflockten Partikeln, die sich gesetzt hatten, dekantiert und dann 10-fach in einem Millipore-Pellicon-System konzentriert.

Eine IL-2-Rezeptoraffinitätssäule wurde vorbereitet, indem 19 ml (gepacktes Volumen) des oben beschriebenen immobilisierten IL-2R-Gels in eine Amicon G-16 x 250 Säule, die mit zwei Adaptoren versehen war, gepackt wurde. Die Säule wurde mit PBS äquilibriert. Dann wurden 160 ml des konzentrierten E. coli-Extraktes von 10 g Zellen auf die Säule aufgetragen. Die Säule wurde bei einer Durchflussgeschwindigkeit von 4 ml/Min laufengelassen. Das Eluat wurde mit einem Gilson 111B UV-Detektor, der mit einem Kippen-Zonen-Aufnahmegerät (Gilson Medical Electronics. Inc., Middletown, Wisconsin, U.S.A.) verbunden war, überwacht.

Die Säule wurde mit 5 Säulenbettvolumen PBS, bzw. bis die Absorption bei 280 nm auf die Grundlinie zurückgekehrt war, gewaschen. Das gebundene rekombinante IL-2 wurde dann, unter Verwendung von 1-2 Säulenbettvolumen 0,2 N Essigsäure, enthaltend 0,2 M NaCl, von der Säule desorbiert. Die gesammelte IL-2-Fraktion wurde, unter Rührend, in einem Amicon-Konzentrator, der mit einer YM 5-Membran ausgerüstet war, konzentriert.

Die IL-2-Aktivität und die Menge Protein in den verschiedenen Fraktionen des Reinigungsverfahrens wurden bestimmt. Die Resultate sind in Tabelle 1 gezeigt.

Tabelle 1

Reinigung von rekombinantem IL-2 aus E. coli

| Fraktion | Volumen (ml) | Protein (mg) | Aktivität (Einheiten x $10^{-6}$) | Aktivitäts-Ausbeute (%) | Spezifische Aktivität (Einheiten/mg x $10^{-7}$) |
|---|---|---|---|---|---|
| Extrakt | 160 | 80,0 | 416 | 100 | 0,52 |
| Durchfluss | 170 | 61,4 | 250 | 60 | 0,41 |
| vereinigte Fraktionen des spezifisch gebundenen Materials | 16 | 11,2 | 179 | 43 | 1,60 |

In Tabelle 1 wurde der ursprüngliche Extrakt verglichen mit dem Material, das nicht an die Säule gebunden hatte (Durchfluss) und mit dem IL-2, das spezifisch an die Säule gebunden hatte, das aber mit der Essigsäurelösung eluiert werden konnte. Die Daten in Tabelle 1 zeigen, dass nur etwa 43% der IL-2-Aktivität im Guanidin•HCl-Extrakt der unlöslichen Fraktion des Bakterienlysates spezifisch an die Säule gebunden wurde, währenddem der Rest der aufgetragenen Aktivität die Säule passierte ohne gebunden zu werden.

Analyse des Durchflusses mittels SDS Polyacrylamid-Gelelektrophorese (Fig. 5. Spur 2) zeigte, dass das IL-2, das darin enthalten war, primär in aggregierter Form, mit hohem Molekulargewicht vorlag. Dadurch, dass der verdünnte Extrakt, der oben beschrieben ist, während mehreren Tagen, anstelle von nur 24 Stunden, stehengelassen wurde, erhöhte sich andererseits der Prozentsatz des monomeren IL-2. Wenn der so behandelte Extrakt auf die IL-2-Rezeptoraffinititässäule aufgetragen wurde, wurde mehr als 90% der gesamten IL-2-Aktivität spezifisch an die Säule gebunden.

6. Reinigung von rekombinantem IL-2 aus Säugerzellen

3 l konditioniertes Medium wurden wie oben beschrieben auf die IL-2-Rezeptoraffinitätssäule aufgetragen. Das rekombinante IL-2 aus Säugerzellen wurde, wie vorher für das rekombinante IL-2 aus E. coli beschrieben, gereinigt.

Die IL-2-Aktivität und die Menge Protein in den verschiedenen Fraktionen des Reinigungsverfahren wurden, wie in Tabelle 2 gezeigt, bestimmt.

Tabelle 2

Reinigung von rekombinantem IL-2 aus konditioniertem Medium von Säugerzellen

| Fraktion | Volumen (ml) | Protein (mg) | Aktivität (Einheiten x $10^{-7}$) | Aktivitäts-Ausbeute (%) | Spezifische Aktivität (Einheiten/mg x $10^{-7}$) |
|---|---|---|---|---|---|
| Ausgangs-medium | 3'000 | 13'500 | 15,5 | 100 | n.b. |
| Durchfluss | 3'000 | 13'500 | 0,5 | 3 | n.b. |
| vereinigte Fraktionen des spezifisch gebundenen Materials | 14 | 7,9 | 13,8 | 89 | 1,8 |

n.b. bedeutet, dass die spezifischen Aktivitätswerte für das Ausgangsmaterial und für das Material, das nicht an die Säule gebunden hatte, nicht bestimmt wurde. Solche Werte würden, wegen dem hohen Serumgehalt in den Zellkulturen, nicht sinnvoll sein.

Die Daten von Tabelle 2 zeigen, dass der grösste Teil des IL-2 im konditionierten Medium spezifisch an die Affinitätssäule gebunden hatte. Die Gesamtausbeute der Aktivität betrug fast 90%. Dieser hohe Prozentsatz von Bindung und Ausbeute, im Vergleich zum bakteriell produzierten rekombinanten IL-2, scheint die Folge einer viel geringeren Aggregatbildung im Säugerzellkultur-Expressionssystem zu sein.

7. Analyse des gereinigten IL-2

Um den Grad der Aggregation des rekombinanten IL-2, das aus den vereinigten Fraktionen des spezifisch gebundenen Materials erhalten wurde, zu bestimmen, wurde das rekombinante IL-2 aus Säugerzellen und das bakterielle rekombinante IL-2 einer Gelfiltration durch Sephadex® G-50 superfein unterworfen. Eine Pharmacia K 26/40-Säule wurde bis zu einer Säulenbetthöhe von 35 cm gepackt. Die Säule wurde mit 50 mM Natriumacetat, pH 3,5, enthaltend 200 mM NaCl und 5 mg/ml Mannit (ICI Americas, Inc., Wilmington, Delaware, U.S.A.), äquilibriert.

Die vereinigten Fraktionen des spezifisch gebundenen Materials (Volumen: 14-16 ml) wurden, vor dem Auftragen auf die Säule, auf 3 ml konzentriert. Die Säulen wurden bei einer Durchflussrate von 0,5 ml/Min, unter Verwendung des oben erwähnten Aequilibrationspuffers, laufen gelassen. 10 Minuten-Fraktionen wurden, unter Verwendung eines LKB-Ultra Rac-7000 Fraktionensammlers (LKB-Produkter, Brommer, Schweden) gesammelt. Der Durchfluss wurde mit einem Gilson Model 111B UV-Detektor, der an ein Kippen-Zonen-Aufnahmegerät angeschlossen war, verfolgt. Die Resultate sind in Figuren 2 (rekombinantes IL-2 aus E. coli) und 3 (rekombinantes IL-2 aus Säugerzellen) gezeigt.

Figuren 2 und 3 zeigen, dass der grösste Teil des rekombinanten IL-2, als eine Spitze, die um Fraktion 83 zentriert war, eluiert wurde. Dieses Material eluierte an einer Position des Elutionsprofils, an der monomeres IL-2 erwartet wird. In beiden Figuren ist nur ein sehr kleiner Teil des Materials mit hohem Molekulargewicht sichtbar (Spitze bei etwa Fraktion 57). Demzufolge geht rekombinantes, humanes IL-2, unabhängig davon ob es in bakteriellen oder in Säugerzellexpressionssystemen hergestellt worden ist, in im wesentlichen unaggregierter, monomerer Form aus der IL-2-Rezeptoraffinitätssäule hervor. In markantem Gegensatz dazu, enthält das bakteriell hergestellte, rekombinante IL-2, das in einer Antikörperaffinitätssäule gereinigt worden ist, ein hohes Mass an aggregiertem Material.

Ein monoklonaler Antikörper der Maus, mit der Bezeichnung 5B1, der gegen ein Dodecapeptid mit der

Aminosäuresequenz der 12 N-terminalen Aminosäurereste des menschlichen IL-2 gerichtet ist, wurde wie von Chizzonite in der Europäischen Patentanmeldung mit der Publikationsnummer 238971, die am 30. September 1987 publiziert wurde, beschrieben, hergestellt. Der monoklonale Antikörper 5B1 wurde aus BALB/c Mausaszitesflüssigkeit, unter Verwendung von Ammoniumsulfatpräzipitation, gefolgt von einer Anionenaustauschchromatographie, wie von Staehelin et al., J. Biol. Chem. 256, 9750-9754 (1981) beschrieben, gereinigt.

Der gereinigte Antikörper wurde auf Nugel P-AF® Poly-N-hydroxysuccinimid, wie oben für die Kopplung von IL-2R beschrieben, immobilisiert. Die Antikörperbeladung betrug 11,8 mg/ml Gel. Eine 3,2 x 13 cm Säule, die mit diesem Antikörperaffinitätsadsorbens gepackt war, wurde zur Reinigung von rekombinantem, menschlichem IL-2, das in E. coli exprimiert worden war, verwendet, wobei das gleiche Protokoll, wie es oben für die Reinigung von IL-2 in der Rezeptoraffinitätssäule beschrieben ist, verwendet wurde.

Rekombinantes IL-2, das mit dieser Säule gereinigt worden war, wurde, wie oben beschrieben, einer Gelfiltration auf Sephadex® G-50 unterzogen. Figur 4 zeigt, dass eine grosse Menge des mit dem Antikörperaffinitätsadsorbens gereinigten IL-2 in einer Form mit hohem Molekulargewicht (als Spitze 1 identifiziert) d.h. in aggregierter, oligomerer Form, eluiert wurde. Spitze 2 ist das nicht-aggregierte monomere IL-2 .

Um zu bestätigen, dass das Material, das von der IL-2-Rezeptoraffinitätssäule gebunden und wieder eluiert worden war, in der Tat IL-2 war, wurden Proben der vereinigten Fraktionen des spezifisch gebundenen Materials einer Aminosäurezusammensetzungsanalyse unterworfen. Die Resultate sind in Tabelle 3 gezeigt.

Tabelle 3

Aminosäurezusammensetzungen von gereinigten rekombinanten IL-2s

| Aminosäure | Reste in IL-2 von E. Coli* | Reste in IL-2 von Säugerzellen | Erwartet |
|---|---|---|---|
| Asparaginsäure | 12,6 | 11,7 | 12 |
| Threonin | 12,5 | 12,3 | 13 |
| Serin | 7,2 | 7,3 | 8 |
| Glutaminsäure | 18,5 | 17,2 | 18 |
| Prolin | 5,1 | 3,9 | 5 |
| Glycin | 2,3 | 3,0 | 2 |
| Alanin | 5,5 | 4,7 | 5 |
| Cystein | 3,0 | 3,4 | 3 |
| Valin | 4,0 | 3,7 | 4 |
| Methionin | 4,5 | 3,9 | 4 |
| Isoleucin | 8,0 | 8,1 | 9 |
| Leucin | 22,1 | 23,4 | 22 |
| Tyrosin | 3,0 | 3,7 | 3 |
| Phenylalanin | 6,9 | 6,5 | 6 |
| Histidin | 3,1 | 3,1 | 3 |
| Lysin | 10,3 | 11,3 | 11 |
| Arginin | 4,0 | 4,2 | 4 |
| Tryptophan | 0,9 | 1,3 | 1 |
| Total | 133 | 133 | 133 |

* Mittel von zwei Analysen

Wie Tabelle 3 gezeigt, besteht bei der Aminosäurezusammensetzung der beiden gereinigten, rekombinanten IL-2s eine gute Uebereinstimmung mit den erwarteten Werten für IL-2.

Zur Abschätzung der Reinheit, des mit dem Rezeptoraffinitätsadsorbens gereinigten rekombinanten IL-2, wurde eine SDS-Polyacrylamid-Gelelektrophorese durchgeführt, deren Resultat in Fig. 5 gezeigt ist. Die elektrophoretische Analyse wurde unter nicht-reduzierenden Bedingungen (d.h. ohne ß-Mercaptoethanol) durchgeführt. Um die Proteine sichtbar zu machen wurden die Gele mit Coomassie-Blau gefärbt.

Spur S in Fig. 5 zeigt eine Mischung von Molekulargewichtsmarkierproteinen. Die Molekulargewichte der Markierproteine sind in Kilodaltons (1 Kd =1000 Daltons) auf der rechten Seite des Gels gezeigt. Folgende Markierproteine wurden verwendet: Phosphorylase B (94 Kd), Rinderserumalbumin (67 Kd), Ovalbumin (43 Kd), Carboanhydrase (30 Kd), Sojabohnentrypsininhibitor (21 Kd) und Lysozym (14,3 Kd). Spuren 1-5 enthalten 20 µg Protein des Gesamtextraktes von rekombinantem, IL-2-produzierenden E. coli;

Durchfluss der Reinigung des E. coli-Extraktes in der Rezeptoraffinitätssäule; an der Rezeptoraffinitätssäule gereinigtes rekombinantes IL-2 aus dem E. coli-Extrakt; konditioniertes Medium der transfizierten Säugerzellen, die rekombinantes IL-2 produzieren bzw. an der Rezeptoraffinitätssäule gereinigtes rekombinantes IL-2 aus konditioniertem Medium.

Fig. 5 zeigt, dass durch Verwendung eines einmaligen Rezeptoraffinitätsreinigungsschrittes, das rekombinante IL-2 aus dem rohen bakteriellen Extrakt soweit gereinigt werden kann, dass im wesentlichen eine Haupt-Bande und eine schwächere, weniger rasch wandernde Bande auf dem Gel sichtbar wird (Spur 3). Die Figur zeigt auch, dass bei Verwendung des gleichen einmaligen Affinitätsreinigungsschrittes, das rekombinante IL-2 aus konditioniertem Medium von Säugerzellen soweit gereinigt werden kann, dass eine Haupt-Bande und eine schneller wandernde, schwächere Bande auf dem Gel sichtbar wird (Spur 5). Western Blot-Analyse zeigte, dass alle in Spuren 3 und 5 beobachtenden Banden, IL-2 enthielten. Die Western-Blot-Analyse wurde, wie von Burnette [Anal. Biochem. 112, 195-203 (1981)] und von Towbin et al. [Proc. Natl. Acad. Sci USA 76, 4350-4354 (1979)] beschrieben, durchgeführt. Als Antikörper wurden die monoklonalen Antikörper 13A6 und 17A1 (spezifisch für glycosyliertes und nicht-glycosyliertes IL-2) und 5B1 (nur spezifisch für nicht-glycosyliertes IL-2) verwendet, die in der Europäischen Patentanmeldung mit der Publikationsnummer 238971 beschrieben sind.

8. Rezeptorafinitätsreinigung von IL-2-Analogen

Plasmide, die Analoge des rekombinanten, menschlichen IL-2 kodieren, wurden unter Verwendung von Stellen-spezifischer Mutagenese (site-specific mutagenesis) und Methoden der DNA-Rekombinationstechnologie hergestellt und in E. coli, wie von Ju et al. [supra] beschrieben, exprimiert. In einem dieser Analoga, wurde das Amino-terminale Alanin weggelassen und der natürliche Cysteinrest an Position 125 durch Serin ersetzt ([des-$Ala^1$-$Ser^{125}$]-IL-2). In einem anderen Analoga, wurde der natürliche Asparaginsäurerest an Position 20 durch Lysin ersetzt ([$Lys^{20}$]-IL-2). Die spezifische biologische Aktivität des rekombinanten [des-$Ala^1$-$Ser^{125}$]- und [des-$Lys^{20}$]-IL-2 betrug 1,7 x $10^7$ bzw. 2,2 x $10^4$ Einheiten/mg Protein, nachdem diese, wie oben beschrieben, gereinigt worden waren.

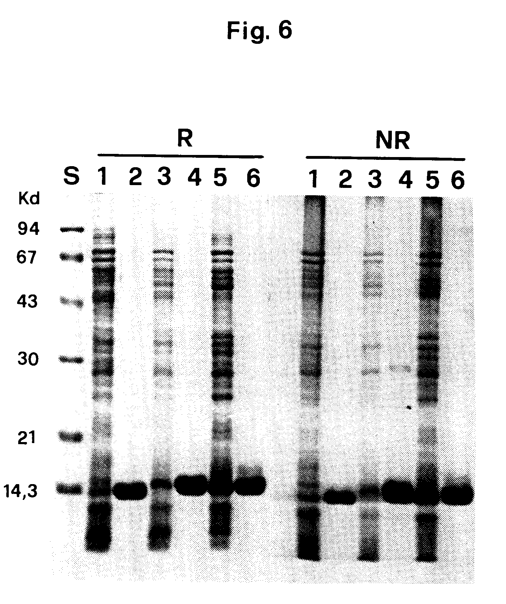

Das [des-$Ala^1$-$Ser^{125}$] IL-2 und das [$Lys^{20}$]-IL-2wurden in der IL-2-Rezeptor-Affinitätssäule, wie oben beschriebenen, gereinigt und die vereinigten Fraktionen des spezifisch gebundenen Materials wurden mittels SDS-Polyacrylamidgelelektrophorese, wie oben beschrieben, analysiert. Die Resultate sind in Fig. 6 gezeigt. Spur S: Mischung von Molekulargewichtsmarkierproteinen; Spur 1: E. coli-Extrakt enthaltend [des-$Ala^1$-$Ser^{125}$]-IL-2; Spur 2: [des-$Ala^1$-$Ser^{125}$]-IL-2 das am erfindungsgemässen Affinitätsadsorbens gereinigt worden ist; Spur 3: E. coli-Extrakt enthaltend [$Lys^{20}$]-IL-2; Spur 4: [$Lys^{20}$]-IL-2 das am erfindungsgemässen Affinitätsadsorbens gereinigt worden ist; Spur 5: E. coli-Extrakt enthaltend Wildtyp rekombinantes, menschliches IL-2 und Spur 6: Wildtyp rekombinantes, menschliches IL-2 das am erfindungsgemässen Affinitätsadsorbens gereinigt worden ist. Die IL-2 enthaltenden Proben wurden unter reduzierenden (R) und nicht-reduzierenden (NR) Bedingungen, d.h. mit oder ohne Reduktion mit ß-Mercaptoethanol, laufen gelassen.

Fig. 6 zeigt, dass das erfindungsgemässe IL-2-Rezeptoraffinitätsabsorbens zur Reinigung von Wildtyp rekombinantem IL-2 und von Varianten des IL-2 verwendet werden kann. Diese IL-2-Varianten unterscheiden sich vom Wildtyp IL-2 durch Deletionen und/oder Substitutionen in der Aminosäuresequenz und können unterschiedliche spezifische biologische Aktivitäten aufweisen. Für alle diese IL-2 gilt, dass das am erfindungsgemässen Affinitätsadsorbens gereinigte Produkt, in im wesentlichen nicht aggregierter, monomerer und hochaktiver Form, vorliegt.

## Patentansprüche

**1.** Ein IL-2 Rezeptor-Affinitätsadsorbens, das lösliche IL-2 Rezeptormoleküle enthält, die an einem festen Träger immobilisiert sind, wobei die immobilisierten Rezeptormoleküle fähig sind, IL-2 spezifisch zu binden.

**2.** Ein Verfahren zur Herstellung eines IL-2 Rezeptor-Affinitätsadsorbens gemäss Anspruch 1, dadurch gekennzeichnet, dass lösliche IL-2 Rezeptormoleküle an einem festen Träger immobilisiert werden.

**3.** Ein Verfahren zur Reinigung von IL-2 aus einer IL-2 haltigen Lösung bzw. einem konditionierten Kulturmedium, das von menschlichen oder tierischen Zellen produziertes IL-2 enthält dadurch gekennzeichnet, dass

a) diese Lösung bzw. das konditionierte Medium, mit einem IL-2 Rezeptor-Affinitätsadsorbens gemäss Anspruch 1 in Kontakt gebracht wird;

b) das Adsorbens zwecks Entfernung ungebundener, verunreinigender Substanzen gewaschen wird;
c) das IL-2 vom gewaschenen Adsorbens desorbiert wird und
d) das Rezeptor-Affinitätsadsorbens vom desorbierten IL-2 abgetrennt wird.

**4.** Ein Verfahren zur Reinigung von rekombinantem IL-2, das in einem bakteriellen Expressionssystem produziert worden ist, dadurch gekennzeichnet, dass
a) kultivierte, bakterielle Zellen, die mit einem Vektor transformiert sind, der fähig ist, IL-2 zu exprimieren, aufgebrochen werden und ein Zell-Lysat hergestellt wird;
b) die unlösliche Fraktion des Zell-Lysats isoliert wird;
c) die isolierte, unlösliche Fraktion des Zell-Lysats mit einer Lösung von etwa 4-7 M Guanidin•HCl extrahiert wird, wodurch ein IL-2 enthaltender Extrakt erhalten wird;
d) dieser Extrakt mit einer Guanidin•HCl-freien Lösung etwa 10- bis 1000-fach verdünnt wird;
e) der verdünnte Extrakt mit einem IL-2 Rezeptor-Affinitätsadsorbens gemäss Anspruch 1 in Kontakt gebracht wird;
f) das Adsorbens zwecks Entfernung ungebundener, kontaminierender Substanzen gewaschen wird;
g) das IL-2 vom gewaschenen Adsorbens desorbiert wird und
h) das Rezeptor-Affinitätsadsorbens vom desorbierten IL-2 abgetrennt wird.

**5.** Ein Verfahren gemäss Anspruch 4, bei dem der Extrakt von Schritt c) etwa 30- bis 50-fach verdünnt wird.

**6.** Das Verfahren gemäss Anspruch 4 oder 5, wobei dem Extraktionsschritt c) ein Präextraktionsschritt vorausgeht, der dadurch gekennzeichnet ist, dass
(i) eine gepufferte Salzlösung und
(ii) eine Lösung von etwa 1,75 bis 2 M Guanidin•HCl
verwendet wird.

**7.** Ein Verfahren gemäss einem der Ansprüche 4-6, bei dem der verdünnte Extrakt von Schritt d) während einer Zeitdauer von etwa 4 Stunden bis mehr als 3 Tagen, stehengelassen wird.

**8.** Ein Verfahren gemäss einem der Ansprüche 4-6 bei dem der verdünnte Extrakt von Schritt d) während einer Zeitdauer von etwa 1 bis 3 Tagen stehengelassen wird.

**9.** Verwendung eines IL-2 Rezeptor-Affinitätsadsorbens gemäss Anspruch 1 zur Reinigung von IL-2.

## Claims

**1.** An IL-2 receptor-affinity adsorbent comprising soluble IL-2 receptor molecules immobilized on a solid support, which immobilized receptor molecules are capable of specifically binding to IL-2.

**2.** A process for the preparation of an IL-2 receptor-affinity adsorbent according to claim 1, characterized in that soluble IL-2 receptor molecules are immobilized on a solid support.

**3.** A process for purifying IL-2 from a solution or a conditioned medium containing IL-2 produced by human or animal cells, characterized by
(a) contacting said solution or conditioned medium with an IL-2 receptor-affinity adsorbent according to claim 1;
(b) washing the adsorbent to remove unbound contaminating substances;
(c) desorbing the IL-2 from the washed adsorbent and
(d) separating the receptor-affinity adsorbent from the desorbed IL-2.

**4.** A process for purifying recombinant IL-2 produced in a bacterial expression system, characterized by
(a) disrupting cultured bacterial cells transformed by a vector capable of directing the expression of IL-2 and producing a cell lysate;
(b) isolating the insoluble fraction from the cell lysate;
(c) extracting the isolated insoluble fraction from the cell lysate with a solution of from about 4-7M guanidine•HCl to obtain an IL-2 containing extract;
(d) diluting this extract by about 10- to 1000-fold with a guanidine•HCl free solution;

(e) contacting the diluted extract with an IL-2 receptor-affinity adsorbent according to claim 1;
(f) washing the adsorbent to remove unbound contaminating substances;
(g) desorbing the IL-2 from the washed adsorbent and
(h) separating the receptor-affinity adsorbent from the desorbed IL-2.

**5.** A process according to claim 4, in which the extract of step (c) is diluted about 30- to 50-fold.

**6.** The process according to claim 4 or 5, whereby the extraction step (c) is preceded by a preextraction step which is characterized in that
(i) a buffered salt solution; and
(ii) a solution of from about 1.75 to 2M guanidine•HCl
is used.

**7.** A process according to any one of claims 4-6, in which the diluted extract from step (d) is allowed to stand for a period of time of about 4 hours to more than 3 days.

**8.** A process according to any one of claims 4-6, in which the diluted extract from step (d) is allowed to stand for about 1 to 3 days.

**9.** Use of an IL-2 receptor-affinity adsorbent in accordance with claim 1 for the purification of IL-2.

**Revendications**

**1.** Adsorbant d'affinité avec récepteur d'IL-2 qui contient des molécules de récepteur d'IL-2 solubles qui sont immobilisées sur un support solide, où les molécules de récepteur immobilisées sont aptes à lier spécifiquement IL-2.

**2.** Procédé de préparation d'un adsorbant d'affinité avec un récepteur d'IL-2 selon la revendication 1, caractérisé en ce qu'on immobilise des molécules de récepteur d'IL-2 solubles sur un support solide.

**3.** Procédé de purification d'IL-2 à partir d'une solution contenant de l'IL-2 ou d'un milieu de culture conditionné qui contient de l'IL-2 produite par des cellules humaines ou animales, caractérisé en ce que :
(a) on met cette solution, ou le milieu conditionné, en contact avec un adsorbant d'affinité avec un récepteur d'IL-2 selon la revendication 1 ;
(b) on lave l'adsorbant afin d'enlever les substances contaminantes non liées ;
(c) on désorbe l'IL-2 de l'adsorbant lavé et
(d) on sépare l'adsorbant d'affinité avec le récepteur d'avec l'IL-2 désorbée.

**4.** Procédé de purification d'IL-2 recombinante qui a été produite dans un système d'expression bactérien, caractérisé en ce que :
(a) on rompt les cellules bactériennes cultivées qui sont transformées avec un vecteur apte à exprimer IL-2, et on prépare un produit de lyse cellulaire ;
(b) on isole la fraction insoluble du lysat cellulaire ;
(c) on extrait la fraction insoluble isolée du lysat cellulaire avec une solution de chlorhydrate de guanidine environ 4-7 M, ce qui permet d'obtenir un extrait contenant l'IL-2 .
(d) on dilue cet extrait environ 10 à 1000 fois avec une solution dépourvue de chlorhydrate de guanidine ;
(e) on met l'extrait dilué en contact avec un adsorbant d'affinité avec récepteur d'IL-2 selon la revendication 1 ;
(f) on lave l'adsorbant afin d'enlever les substances contaminantes non liées ;
(g) on désorbe l'IL-2 de l'adsorbant lavé ; et
(h) on sépare l'adsorbant d'affinité avec le récepteur d'avec l'IL-2 désorbée.

**5.** Procédé selon la revendication 4, dans lequel l'extrait de l'étape (c) est dilué environ 30 à 50 fois.

**6.** Procédé selon la revendication 4 ou 5, dans lequel l'étape d'extraction (c) est précédée d'une étape de préextraction qui se caractérise en ce que l'on utilise :

(i) une solution de sel tamponnée ; et
(ii) une solution de chlorhydrate de guanidine environ 1,75 à 2 M.

**7.** Procédé selon l'une des revendications 4-6, dans lequel on laisse reposer l'extrait dilué de l'étape (d) pendant une durée d'environ 4 heures à plus de 3 jours.

**8.** Procédé selon l'une des revendications 4-6, dans lequel on laisse reposer l'extrait dilué de l'étape (d) pendant une durée d'environ 1 à 3 jours.

**9.** Application d'un adsorbant d'affinité avec récepteur d'IL-2 selon la revendication 1 à la purification de l'IL-2.

Fig.1

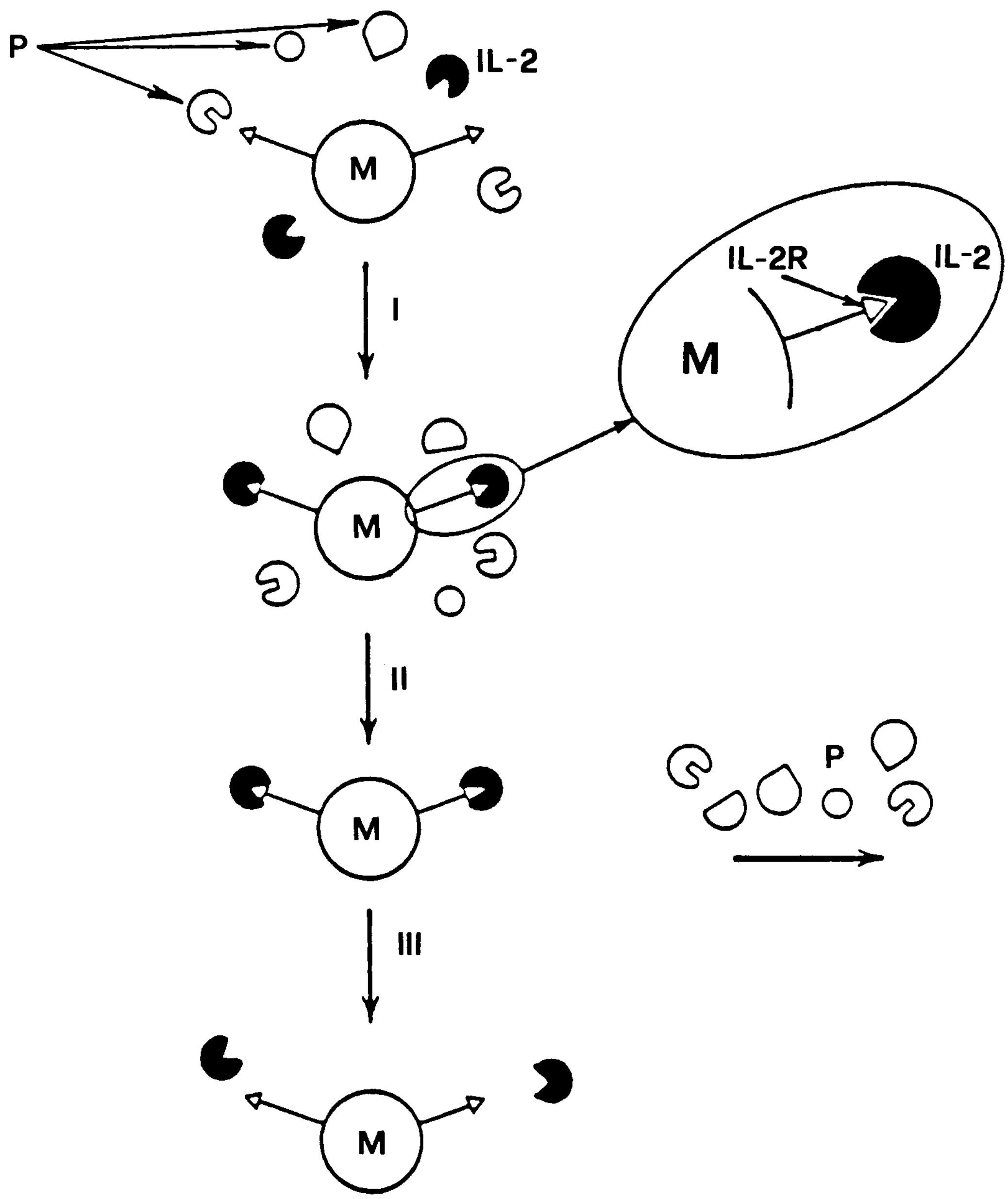

Fig. 2

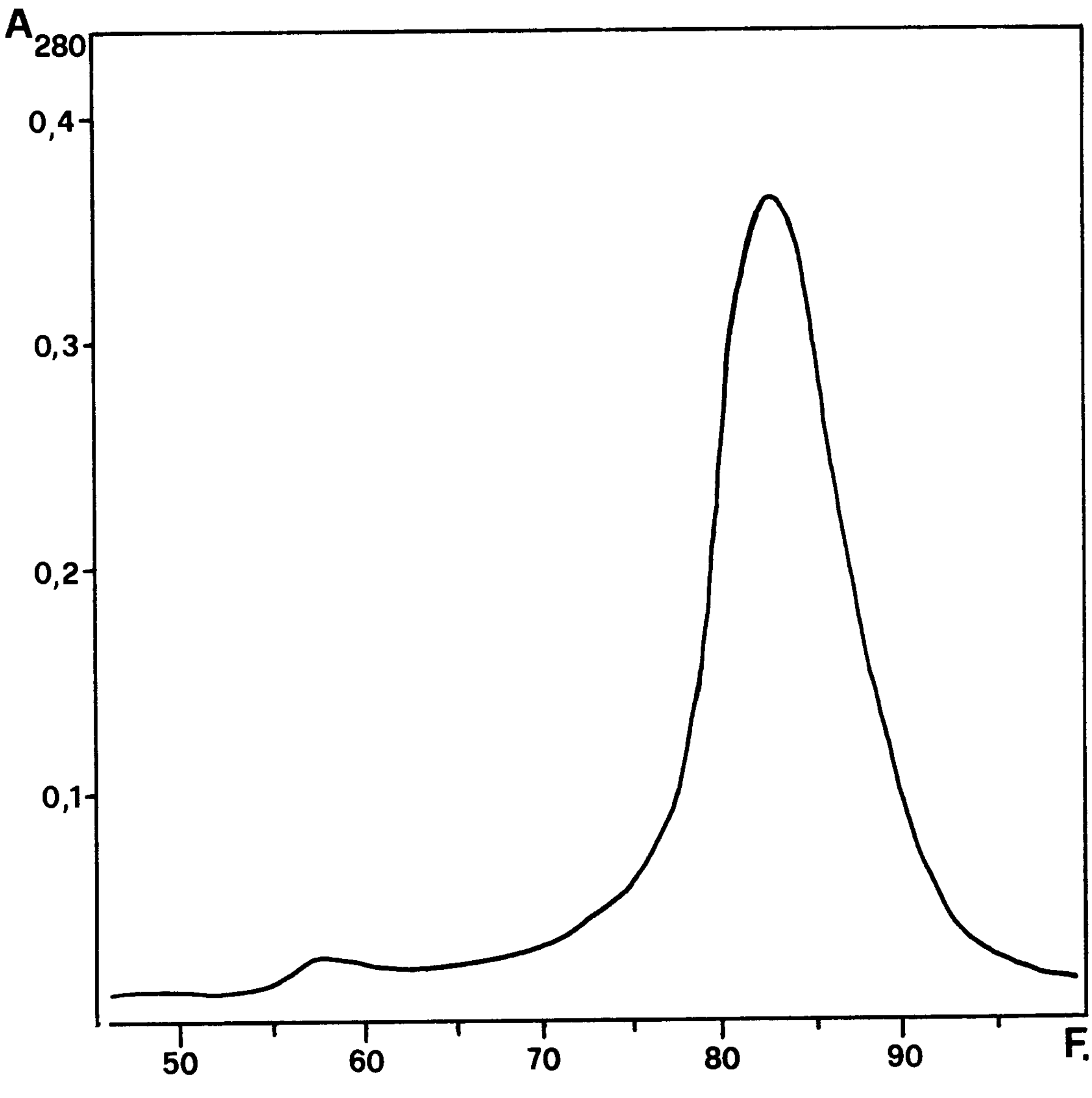

Fig. 3

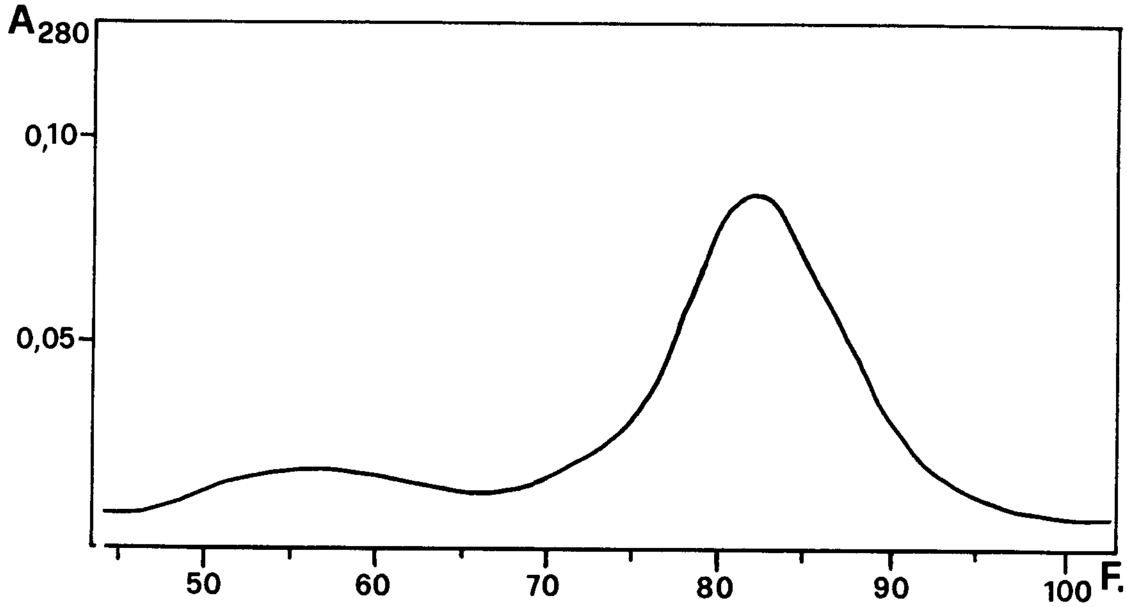

Fig. 4

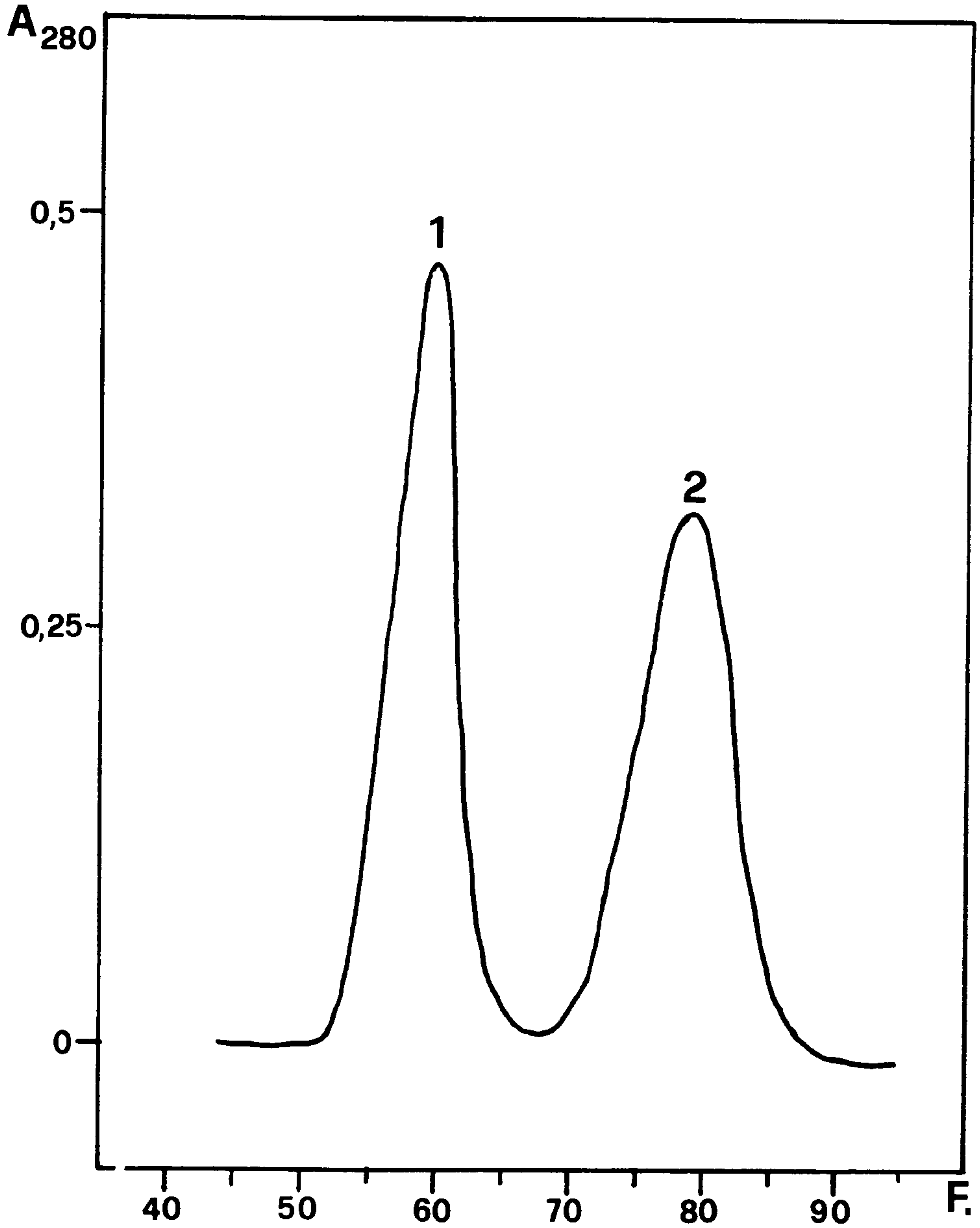

Fig. 5

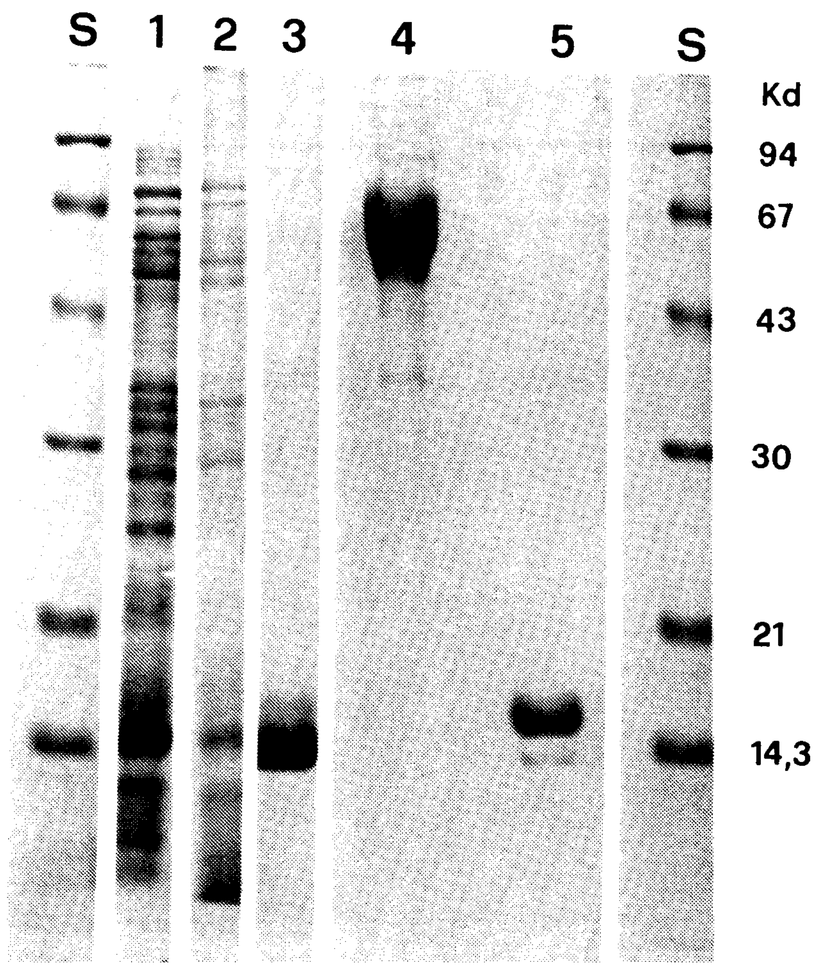

Fig. 6